# EUROPEAN PATENT SPECIFICATION

(11) **EP 3 114 085 B1**
(45) Date of publication and mention of the grant of the patent: **13.02.2019**
(21) Application number: 15758481.4
(22) Date of filing: 09.03.2015
(51) Int. Cl.: C02F 1/28, B01J 20/28, B01J 20/06, C01F 17/00, C12N 1/02, C02F 101/30, C02F 103/06

(54) **METHOD AND DEVICE WITH CERIUM (IV) OXIDE WITH EXCEPTIONAL BIOLOGICAL CONTAMINANT REMOVAL PROPERTIES**
VERFAHREN UND VORRICHTUNG MIT CERIUM-(IV)-OXID MIT AUSSERORDENTLICHEN EIGENSCHAFTEN ZUR BESEITIGUNG BIOLOGISCHER VERUNREINIGUNGEN
PROCÉDÉ ET DISPOSITIF AVEC OXYDE DE CÉRIUM (IV) À PROPRIÉTÉS EXCEPTIONNELLES D'ÉLIMINATION DE CONTAMINANTS BIOLOGIQUES

(30) Priority: 07.03.2014 US 201461949810 P
(43) Date of publication of application: 11.01.2017
(73) Proprietor: Secure Natural Resources LLC, Chicago, IL 60611 (US)
(72) Inventor: PSARAS, Dimitrios, Bound Brook, NJ 08805 (US); GAO, Yuan, Greenwood Village, CO 80111 (US); HANELINE, Mason, Henderson, NV 89074 (US); LUPO, Joseph, Henderson, NV 89052 (US); LANDI, Carol, Devon, PA 19333 (US)
(74) Representative: Balder IP Law, S.L.
(86) International application number: PCT/US2015/019476
(87) International publication number: WO 2015/134976

(56) References cited:
- US-A1- 2011 309 017
- US-A1- 2012 031 827
- US-A1- 2013 212 944
- PATIL ET AL: "Protein adsorption and cellular uptake of cerium oxide nanoparticles as a function of zeta potential", BIOMATERIALS, ELSEVIER SCIENCE PUBLISHERS BV., BARKING, GB, vol. 28, no. 31, 18 August 2007 (2007-08-18), pages 4600-4607, XP022207000, ISSN: 0142-9612, DOI: 10.1016/J.BIOMATERIALS.2007.07.029

## Description

### BACKGROUND

Various technologies have been used to remove biological contaminants from aqueous systems. Examples of such techniques include adsorption on high surface area materials, such as alumina and the use of highly oxidative materials such as chlorine and bromine. The more successful techniques that have been used in large municipal water supplies are not practical for residential applications because of space requirements and the need to use dangerous chemicals. The two most common techniques for residential water treatment have been filtration and chlorination. U.S. Patent Application Publications 2011/0309017 and 2012/0031827 disclose the use of cerium (IV) oxide compositions to remove biological contaminants from aqueous solutions, which defer from those of the present disclosure.

### SUMMARY

This disclosure relates generally to a method and a device employing cerium-containing compositions for removing biological and other target contaminants from aqueous streams as specified in independent claims 1 and 5. More specifically, this disclosure is particularly concerned with cerium-containing compositions for removing biological contaminants from groundwater and drinking water. Typically, the cerium-containing composition is cerium oxide. More typically, the cerium-containing composition can be cerium (IV) oxide. The biological contaminants can be present at high or very low concentrations. The cerium-containing composition can remove the biological contaminants from the aqueous streams when they are present at high or very low concentrations.

It has now been found that biological and other target contaminants can be efficiently and effectively removed from water and other aqueous liquid feed stocks by treating the aqueous stream containing one or more biological contaminants with a cerium-containing composition. The cerium-containing composition generally comprises a cerium (IV) oxide composition (CeO₂). The cerium (IV) oxide composition can be in a crystalline form. Moreover, the cerium (IV) oxide composition can have a high surface area. Surprisingly, it has further been found that using cerium (IV) oxide composition (CeO₂) with particular characteristics as described below enables the capture and removal of biological target contaminants with higher removal capacities compared to traditional removal media, including cerium oxide lacking one or more of these particular characteristics.

In accordance with the invention a method of contacting a cerium (IV) oxide composition with a biological contaminant-containing aqueous is provided. The contacting of the cerium (IV) oxide composition with the biological contaminant-containing aqueous stream can remove some of the biological contaminant from the biological contaminant-containing aqueous stream. In the method the following (i) through (vi) are true:
(i) the cerium (IV) oxide composition has a zeta potential at about pH 7 of no more than about 30 mV and of more than about 1 mV;
(ii) the cerium (IV) oxide composition has a particle size D₁₀ of more than about 0.5 µm and no more than about 7 µm;
(iii) the cerium (IV) oxide composition has a particle size D₅₀ of more than about 2 µm and no more than about 20 µm;
(iv) the cerium (IV) oxide composition has a particle size D₉₀ of more than about 12 µm and no more than about 50 µm;
(v) the cerium (IV) oxide composition has a crystallite size of more than about 1 nm and no more than about 22 nm; and
(vi) the cerium (IV) oxide composition has an acidic site concentration of more than about 0.0001 acidic sites/kg and no more than about 0.020 acidic sites/kg.

According to the invention a device is provided, the device having an inlet to receive an aqueous stream having a first level of a biological contaminant; a contacting chamber, in fluid communication with the inlet and containing a cerium (IV) oxide composition to contact the aqueous stream; and an outlet in fluid communication with the contacting chamber to output the aqueous stream having a second level of the biological contaminant. The aqueous stream has the first level of biological contaminant prior to the of the aqueous stream contacting the cerium (IV) oxide composition and has a second level of biological contaminant after the contacting of the aqueous stream with the cerium (IV) oxide. The first level of biological contaminant is greater than the second level of the biological contaminant. In the device, the following (i) through (vi) are true:
(i) the cerium (IV) oxide composition has a zeta potential at about pH 7 of no more than about 30 mV and of more than about 1 mV;
(ii) the cerium (IV) oxide composition has a particle size D₁₀ of more than about 0.5 µm and no more than about 7 µm;
(iii) the cerium (IV) oxide composition has a particle size D₅₀ of more than about 2 µm and no more than about 20 µm;
(iv) the cerium (IV) oxide composition has a particle size D90 of more than about 12 µm and no more than about 50 µm;
(v) the cerium (IV) oxide composition has a crystallite size of more than about 1 nm and no more than about 22 nm; and
(vi) the cerium (IV) oxide composition has an acidic site concentration of more than about 0.0001 acidic sites/kg and no more than about 0.020 acidic sites/kg.

In some embodiments, the cerium (IV) oxide composition can have a zeta potential from about 7.5 to about 12.5 mV at about pH 7. Moreover in some embodiments, the cerium (IV) oxide composition can have, prior to sorbing the biological contaminant, a zeta potential from about 7.5 to about 12.5 mV at about pH 7.

In some embodiments, the cerium (IV) oxide composition can have a particle size D₁₀ is from about 1 to about 3 µm.

In some embodiments, the cerium (IV) oxide can have a particle size D₅₀ from about 7.5 to about 10.5 µm.

In some embodiments, the cerium (IV) oxide composition can have a particle size D₉₀ from about 20 to about 30 µm.

In some embodiments, the cerium (IV) oxide composition can have a crystallite size from about 7.5 to about 12. 5 nm.

In some embodiments, the cerium (IV) oxide composition can have a number of acid sites from more than about 0.0001 to no more than about 0.020 acidic sites/kg of the cerium (IV) oxide composition.

In some embodiments, the biological contaminant can be selected from the group consisting of bacteria, yeasts, algae, and viruses.

In some embodiments, the biological contaminant can be one selected from the group of Klebsiella oxytoca, Saccharomyces cerevisiae, Selenastum capriocornutum, and MS2.

The cerium (IV) oxide composition can be unsupported or supported. The supported cerium (IV) oxide composition can be deposited on a single support or deposited on multiple supports. The supports can be without limitation alumina, aluminosilicates, ion exchange resins, organic polymers, and clays. The cerium (IV) oxide composition can be deposited and/or mixed with a polymeric porous material. Moreover, it is believed that the cerium (IV) oxide composition surface exposure is enhanced when the cerium (IV) oxide composition is deposited and/or mixed with the polymeric porous material.

These and other advantages will be apparent from the disclosure of the aspects, embodiments, and configurations contained herein.

As used herein, "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

It is to be noted that the term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The term "means" as used herein shall be given its broadest possible interpretation. Accordingly, a claim incorporating the term "means" shall cover all structures, materials, or acts set forth herein, and all of the equivalents thereof. Further, the structures, materials or acts and the equivalents thereof shall include all those described in the summary of the invention, brief description of the drawings, detailed description, abstract, and claims themselves.

Unless otherwise noted, all component or composition levels are in reference to the active portion of that component or composition and are exclusive of impurities, for example, residual solvents or by-products, which may be present in commercially available sources of such components or compositions.

All percentages and ratios are calculated by total composition weight, unless indicated otherwise.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein. By way of example, the phrase from about 2 to about 4 includes the whole number and/or integer ranges from about 2 to about 3, from about 3 to about 4 and each possible range based on real (e.g., irrational and/or rational) numbers, such as from about 2.1 to about 4.9, from about 2.1 to about 3.4, and so on.

### DETAILED DESCRIPTION OF FIGURES

The accompanying drawings, which are incorporated in and constitute a part of the specification, illustrate embodiments of the disclosure and together with the general description of the disclosure given above and the detailed description given below, serve to explain the principles of the disclosure.
Fig. 1 shows the *Klebsiella oxytoca* bacteria with respect to the incubation time for a control and for a cerium (IV) oxide composition according to some embodiments of the present disclosure;
Fig. 2 shows the *Saccharomyces cerevisiae* yeast count with respect to incubation time for a control and for the cerium (IV) oxide composition according to some embodiments of the present disclosure;
Fig. 3 shows the *Selenastum Capriocornutum* count with respect to incubation time for a control and for the cerium (IV) oxide composition according to some embodiments of the present disclosure;
Fig. 4 shows the MS2 Bacteriophage concentration with respect to incubation time for a control and for the cerium (IV) oxide composition according to some embodiments of the present disclosure;
Fig. 5 shows the *Klebsiella oxytoca* count with respect to incubation time for a control, for an oxide of cerium (IV) of the prior art (Comparative Example 1), and for the cerium (IV) oxide composition of according to some embodiments of the present disclosure;
Fig. 6 shows the *Saccharomyces cerevisiae* count with respect to incubation time for a control, for an oxide of cerium (IV) of the prior art (Comparative Example 1), and for the cerium (IV) oxide composition of according to some embodiments of the present disclosure;
Fig. 7 shows the *Selenastum Capriocornutum* count with respect to incubation time with respect to a control, for an oxide of cerium (IV) of the prior art (Comparative Example 1), and for the cerium (IV) oxide composition of according to some embodiments of the present disclosure;
Fig. 8 shows the MS2 Bacteriophage concentration with respect to incubation time for a control, for an oxide of cerium (IV) of the prior art (Comparative Example 1), and for the cerium (IV) oxide composition of according to some embodiments of the present disclosure;
Fig. 9 is a comparison plot of the zeta potential of both the cerium (IV) oxide composition of the Example and a prior art oxide of cerium (IV) against pH; and
Fig. 10 is a comparison plot of the particle size distribution for both the cerium (IV) oxide composition of the Example and a prior art oxide of cerium (IV).

### DETAILED DESCRIPTION

The process of the disclosure is primarily envisioned for removing biological contaminants from an aqueous stream using a cerium (IV) oxide composition (CeO₂) having particular properties. The aqueous stream can be one or more of a drinking water and groundwater source that contains undesirable amounts of biological and/or other contaminants. Furthermore, the aqueous stream can include without limitation well waters, surface waters (such as water from lakes, ponds and wetlands), agricultural waters, wastewater from industrial processes, and geothermal waters.

Generally, the cerium (IV) oxide composition can be used to treat any aqueous stream containing a biological contaminant. The cerium (IV) oxide composition of the present disclosure has a number of properties that are particularly advantageous for biological contaminant removal. Contacting of the cerium (IV) oxide composition with the aqueous stream containing the biological contaminant can effectively reduce biological contaminant level in the aqueous stream. Typically, the contacting of the cerium (IV) oxide composition with the aqueous stream can reduce the biological contaminant level in the aqueous stream by more than about 75%. More typically, the contacting of the cerium (IV) oxide composition with the aqueous stream can reduce the biological contaminant level in the aqueous stream by more than about 80%, more typically more than about 85%, more typically more than about 90%, more typically more than about 95%, more typically more than about 97.5%, and even more typically more than about 99.5%.

The cerium (IV) oxide composition has a zeta-potential, at pH 7, of more than about 1 mV. While not wanting to be bound by any theory it is believed that the zeta of the cerium (IV) oxide composition can affect the removal of the biological contaminant from an aqueous stream. Typically, the cerium (IV) oxide composition has a zeta-potential, at pH 7, of more than about 5 mV. More typically, the zeta-potential, at pH 7, of the cerium (IV) oxide composition is more than about 10 mV. Generally, the cerium (IV) oxide composition has a zeta-potential of no more than about 30 mV. More generally, the zeta-potential of the cerium (IV) oxide composition is no more than about 20 mV or even more typically no more than about 15 mV. At a pH of about 7, the cerium (IV) oxide composition has zeta-potential of no more than one of about 30 mV, about 20 mV and about 15 mV and a zeta-potential of more than one of about 1 mV, about 5 mV, and 10 mV. The zeta-potential of the cerium (IV) oxide composition at pH 7 usually ranges from about 7.5 to about 12.5 mV. It can be appreciated that the cerium (IV) oxide composition can have any one of the described zeta-potentials in combination with any one or more of the below isoelectric points, surface areas, average pore volumes, average pore sizes, particle sizes, crystalline sizes, and number of acidic sites.

Generally, the cerium (IV) oxide composition typically has an isoelectric point of more than about pH 7, more generally of more than about pH 8, and even more generally of more than about pH 9 but generally no more than about pH 12, more generally no more than about pH 11, and even more generally no more than about pH 10. The isoelectric point typically ranges from about pH 8.5 to about pH 10. While not wanting to be bound by any theory it is believed that the isoelectric point of the cerium (IV) oxide composition can affect the removal of the biological contaminant from an aqueous stream. It can be appreciated that the cerium (IV) oxide composition can have any one of the described isolectric points in combination with any one or more of: the above zeta-potentials; and the below surface areas, average pore volumes, average pore sizes, particle sizes, crystalline sizes and number of acidic sites.

The cerium (IV) oxide composition can commonly have a surface area from about 30 to about 200 m²/g, more commonly from about 60 to about 180 m²/g, or even more typically from about 100 to about 150 m²/g. Typically, the surface of the cerium (IV) oxide composition is from about 100 to about 150 m²/g, more typically from about 110 to about 150 m²g/. While not wanting to be bound by any theory it is believed that the surface area of the cerium (IV) oxide composition can affect the removal of the biological contaminant from an aqueous stream. It can be appreciated that the cerium (IV) oxide composition can have any one of the described surface areas in combination with any one or more of: the above zeta-potentials and isoelectric points; and the below average pore volumes, average pore sizes, particle sizes, crystalline sizes and number of acidic sites.

The cerium (IV) oxide composition typically has an average (mean, median, and mode) pore volume (as determined by N₂ adsorption) of more than about 0.01 cm³/g, more typically of more than about 0.1 cm³/g, and more typically of more than about 0.2 cm³/g but typically no more than about 0.85 cm³/g, more typically no more than about 0.8 cm³/g, more typically no more than about 0.75 cm³/g, more typically no more than about 0.65 cm³/g, more typically no more than about 0.6 cm³/g, more typically no more than about 0.55 cm³/g, more typically no more than about 0.5 cm³/g, and even more typically no more than about 0.45 cm³/g. The pore volume can range from about 0.3 to about 0.4 cm³/g, from more than about 0.4 to about 0.5 cm³/g, or from more than about 0.5 to about 0.6 cm³/g. While not wanting to be bound by any theory it is believed that the average pore volume of the cerium (IV) oxide composition can affect the removal of the biological contaminant from an aqueous stream. It can be appreciated that the cerium (IV) oxide composition can have any one of the described average pore volumes in combination with any one or more of: the above zeta-potentials, isoelectric points, and surface areas; and the below average pore sizes, particle sizes, crystalline sizes and number of acidic sites.

The cerium (IV) oxide composition generally has an average (mean, median, and mode) pore size (as determined by the BJH method) of more than about 0.5 nm, more generally of more than about 1 nm, and more generally of more than about 6 nm but generally no more than about 20 nm, more generally no more than about 15 nm, and even more generally no more than about 12 nm. The average pore size can range from about 0.5 to about 6.5 nm, from more than about 6.5 to about 13 nm, or from more than about 13 to about 20 nm. While not wanting to be bound by any theory it is believed that the average pore size of the cerium (IV) oxide composition can affect the removal of the biological contaminant from an aqueous stream. It can be appreciated that the cerium (IV) oxide composition can have any one of the described average pore sizes in combination with any one or more of: the above zeta-potentials, isoelectric points, surface areas and average pore volumes; and the below particle sizes, crystalline sizes and number of acidic sites.

The cerium (IV) oxide composition is usually in particulate form. Typically, the particulate cerium (IV) oxide composition has one or more of a particle size D₁₀, particle size D₅₀ and particle D₉₀. While not wanting to be bound by any theory it is believed that the one or more of a particle size D₁₀, particle size D₅₀ and particle D₉₀ surface area of the cerium (IV) oxide composition can affect the removal of the biological contaminant from an aqueous stream. It can be appreciated that the cerium (IV) oxide composition can have any one of the described particle sizes D₁₀, D₅₀ or D₉₀ in combination with any one or more of: the above zeta-potentials, isoelectric points, surface areas, average pore volumes and average pore sizes; and the below crystalline sizes and number of acidic sites.

The particulate cerium (IV) oxide composition commonly has a particle size D₁₀ of more than 0.5 µm and no more than 7 µm, for example from about 1 to about 3 µm. More commonly, the cerium (IV) oxide composition typically has a particle size D₁₀ of more than about 0.05 µm, even more of more than about 1 µm but more commonly no more than about 5 µm, and yet even more commonly no more than about 3 µm. The particle size D₁₀ typically ranges from about 1 to about 3 µm. While not wanting to be bound by any theory it is believed that the particle size D₁₀ of the cerium (IV) oxide composition can affect the removal of the biological contaminant from an aqueous stream. It can be appreciated that the cerium (IV) oxide composition can have any one of the described D₁₀ particle sizes in combination with any one or more of: the above zeta-potentials, isoelectric points, surface areas, average pore volumes and average pore sizes; and the below crystalline sizes and number of acidic sites.

Moreover, the cerium (IV) oxide composition has a particle size D₅₀ of more than about 2 µm, more generally of more than about 4 µm, and more generally of at least about 5 µm but no more than about 20 µm, generally no more than about 15 µm, and more generally no more than about 12 µm. The particle size D₅₀ usually ranges from about 7.5 to about 10.5 µm. While not wanting to be bound by any theory it is believed that the particle size D₅₀ of the cerium (IV) oxide composition can affect the removal of the biological contaminant from an aqueous stream. It can be appreciated that the cerium (IV) oxide composition can have any one of the described D₅₀ particle sizes in combination with any one or more of: the above zeta-potentials, isoelectric points, surface areas, average pore volumes and average pore sizes; and the below crystalline sizes and number of acidic sites.

The cerium (IV) oxide composition commonly has a particle size D₉₀ of more than about 12 µm, more commonly of more than about 15 µm, and even more commonly of more than about 20 µm but no more than about 50 µm, commonly no more than about 40 µm, and more commonly no more than about 30 µm. The particle size D₉₀ generally ranges from about 20 to about 30 µm. While not wanting to be bound by any theory it is believed that the particle size D₉₀ of the cerium (IV) oxide composition can affect the removal of the biological contaminant from an aqueous stream. It can be appreciated that the cerium (IV) oxide composition can have any one of the described D₉₀ particle sizes in combination with any one or more of: the above zeta-potentials, isoelectric points, surface areas, average pore volumes and average pore sizes; and the below crystalline sizes and number of acidic sites.

The cerium (IV) oxide composition typically has a crystallite size of more than about 1 nm, more typically of more than about 4 nm, and even more typically of more than about 7.5 nm but no more than about 22 nm, typically no more than about 17 nm, and more typically no more than about 12.5 nm. The crystallite size commonly ranges from about 7.5 to about 12.5 nm. While not wanting to be bound by any theory it is believed that the crystallite size of the cerium (IV) oxide composition can affect the removal of the biological contaminant from an aqueous stream. It can be appreciated that the cerium (IV) oxide composition can have any one of the described crystalline sizes in combination with any one or more of the above zeta-potentials, isoelectric points, surface areas, average pore volumes, average pore sizes and particle sizes, and the below number of acidic sites.

The cerium (IV) oxide has no more than about 0.020 acidic sites/kg as measured by a zeta-potential titration. Generally, the cerium (IV) oxide has no more than about 0.015 acidic sites/kg, even more generally no more than about 0.010 acidic sites/kg, yet even more generally no more than about 0.005 acid sites/kg, and even yet more generally no more than about 0.001 acid sites/kg as measured by a zeta-potential titration. Even yet more generally, the cerium (IV) oxide has about 0 to about 0.001 acid sites/kg as measured by a zeta-potential titration. While not wanting to be bound by any theory it is believed that the number of acid sites/kg of the cerium (IV) oxide composition can affect the removal of the biological contaminant from an aqueous stream. It can be appreciated that the cerium (IV) oxide composition can have any one of the described number of acid sites in combination with any one or more of the above zeta-potentials, isoelectric points, surface areas, average pore volumes, average pore sizes and particle sizes.

The level of cerium (IV) oxide, Ce(IV)O₂ in the cerium (IV) oxide composition can vary. The cerium (IV) oxide composition typically comprises more than about 75 wt% Ce(IV)O₂, more typically more than about 85 wt% Ce(IV)O₂, even more typically more than about 90 wt% Ce(IV)O₂, or yet even more typically more than about 99.5 wt% Ce(IV)O₂.

The cerium (IV) oxide composition can contain rare earth oxides other than cerium (IV) oxide. Commonly, the rare earth oxides other than cerium (IV) oxide comprise no more than about 40 wt.%, more commonly no more than about 25 wt.%, and even more commonly no more than about 10 wt.% of the cerium (IV) oxide composition.

Usually, the cerium (IV) oxide composition can contain non-rare earth materials. Generally, the non-rare earth materials typically comprise no more than about 5 wt.%, more generally no more than about 2.5 wt.%, and even more generally no more than about 1 wt.% of the cerium (IV) oxide composition. In some examples, the cerium (IV) oxide composition can be free of any added non-rare materials. That is, the level of non-rare earth materials contained in the cerium (IV) oxide composition typically comprise naturally occurring "impurities" present in cerium oxide. Commonly, any one non-rare material contained in the cerium (IV) oxide composition is no more than about 4 wt%, more commonly no more than about 2.5 wt%, even more commonly no more than about 1 wt% and yet even more commonly no more than about 0.5 wt%.

It can be appreciated that the cerium (IV) oxide composition can have any one or more of the described wt% cerium(IV) oxide, wt% of rare earth oxides other than cerium (IV) oxide, and wt% of non-rare earth materials in combination with any one or more of the above zeta-potentials, isoelectric points, surface areas, average pore volumes, average pore sizes, particle sizes, crystalline sizes, and number of acid sites.

While not wishing to be bound by any theory, it is believed that the difference between one or more the zeta-potential, isoelectric point, surface area, an average (mean, median, and mode) pore volume (as determined by N₂ adsorption), an average (mean, median, and mode) pore size (as determined by the BJH method), D₁₀ particle size, D₅₀ particle size, D₉₀ particle size, crystallite size and number of acidic sites/kg of the cerium (IV) oxide of the present disclosure and oxides of cerium of the prior art better enables biological contaminant to contact reaction sites in the cerium (IV) oxide composition and be removed from the biological-contaminant-containing aqueous stream by the cerium (IV) oxide composition.

In some examples, the biological contaminant-containing aqueous stream is passed through an inlet into a vessel at a temperature and pressure, usually at ambient temperature and pressure, such that the water in the biological contaminant-containing aqueous stream remains in the liquid state. In this vessel the biological contaminant-containing aqueous stream is contacted with the cerium (IV) oxide composition. The contacting of the cerium (IV) oxide with the the biological contaminant-containing aqueous stream leads to the biological contaminant one or more of sorbing and reacting with the cerium (IV) oxide composition. The one or more of sorbing and reacting of the cerium (IV) oxide composition with the biological contaminant removes the biological contaminant from the biological contaminant-containing aqueous stream.

In some examples, the cerium (IV) oxide composition can be deposited on a support material. Furthermore, the cerium (IV) oxide can be deposited on one or more external and/or internal surfaces of the support material. It can be appreciated that persons of ordinary skill in the art generally refer to the internal surfaces of the support material as pores. The cerium (IV) oxide composition can be supported on the support material with or without a binder. In some embodiments, the cerium (IV) oxide composition can be applied to the support material using any conventional techniques such as slurry deposition.

In some examples, the cerium (IV) oxide composition is slurried with the biological contaminant-containing aqueous stream. It can be appreciated that the cerium (IV) oxide composition and the biological contaminant-containing aqueous stream are contacted when they are slurried. While not wanting to be bound by any theory, it is believed that some, if not most or all of the biological contaminant contained in the biological contaminant-containing aqueous stream is removed from the biological contaminant-containing aqueous stream by the slurring and/or contacting of the cerium (IV) oxide composition with the biological contaminant-containing aqueous stream. Following the slurring and/or contacting of the cerium (IV) oxide with the biological contaminant-containing aqueous stream, the slurry is filtered by any known solid liquid separation method. The term "some" refers to removing no more than about 50% of the biological contaminant contained in the aqueous stream. More generally, the term "some" refers to one or more of removing no more than about 10%, no more than about 20%, no more than about 30%, and no more than about 40% of the biological contaminant contained in the aqueous stream. The term "most" refers to removing more than about 50% but no more than about 100% of the biological contaminant contained in the aqueous stream. More commonly, the term "most" refers to one or more of removing more than about 60%, more than about 70%, more than about 90%, and more than about 90% but no more than 100% of the biological contaminant contained in the aqueous stream. The term "all" refers to removing about 100% of the biological contaminant contained in the aqueous stream. More generally, the term "all" refers to removing more than 98%, 99%, 99.5%, and 99.9% of the biological contaminant contained in the aqueous stream.

In some examples, the cerium (IV) oxide composition is in the form of a fixed bed. Moreover, the fixed bed of cerium (IV) oxide is normally comprises cerium (IV) oxide in the form of cerium (IV) oxide particles. The cerium (IV) oxide particles can have a shape and/or form that exposes a maximum cerium (IV) oxide particle surface area to the aqueous liquid fluid with minimal back-pressure and the flow of the aqueous liquid fluid through the fixed bed. However, if desired, the cerium (IV) oxide particles may be in the form of a shaped body such as beads, extrudates, porous polymeric structures or monoliths. In some embodiments, the cerium (IV) oxide composition can be supported as a layer and/or coating on such beads, extrudates, porous polymeric structures or monolith supports.

The contacting of the cerium (IV) oxide composition with the biological contaminant-containing aqueous stream normally takes place at a temperature from about 4 to about 100 degrees Celsius, more normally from about 5 to about 40 degrees Celsius. Furthermore, the contacting of cerium (IV) oxide with the biological contaminant-containing stream commonly takes place at a pH from about pH 1 to about pH 11, more commonly from about pH 3 to about pH 9. The contacting of the cerium (IV) oxide composition with biological contaminant-containing aqueous stream generally occurs over a period of time of more than about 1 minute and no more than about 24 hours.

The nature and objects of the disclosure are further illustrated by the following example, which is provided for illustrative purposes only and not to limit the disclosure as defined by the claims.

The following examples are provided to illustrate certain aspects, embodiments, and configurations of the disclosure and are not to be construed as limitations on the disclosure, as set forth in the appended claims. All parts and percentages are by weight unless otherwise specified.

### EXAMPLE

A cerium (IV) oxide composition was prepared by the following method. In a closed, stirred container a one liter of a 0.12 M cerium (IV) ammonium nitrate solution was prepared from cerium (IV) ammonium nitrate crystals dissolved in nitric acid and held at approximately 90°C for about 24 hours. In a separate container 200 ml of a 3M ammonium hydroxide solution was prepared and held at room temperature. Subsequently the two solutions were combined and stirred for approximately one hour. The resultant precipitate was filtered using Bückner funnel equipped with filter paper. The solids were then thoroughly washed in the Bückner using deionized water. Following the washing/filtering step, the wet hydrate was calcined in a muffle furnace at approximately 450°C for three hours to form the cerium (IV) oxide composition.

The cerium (IV) oxide composition material used had a zeta-potential of about 9.5 mV at a pH of about pH 7, an isoelectric point of about pH 9.1, about 0.001 acidic sites/kg as measured by zeta-potential titration, a surface area between about 110 and about 150 m²/g, a particle size D₁₀ of about 2 µm, a particle size D₅₀ of about 9 µm, a particle size D₉₀ of about 25 µm, and a crystallite size of about 10 nm. The crystallite size, that is the size of the individual crystals, was measured by XRD or TEM. The Dₓₓ particle sizes were measured by laser diffraction; they are the size of the particles that are made up of the individual crystallites.

### Bacterial Removal Characteristics of the Cerium (IV) Oxide Composition

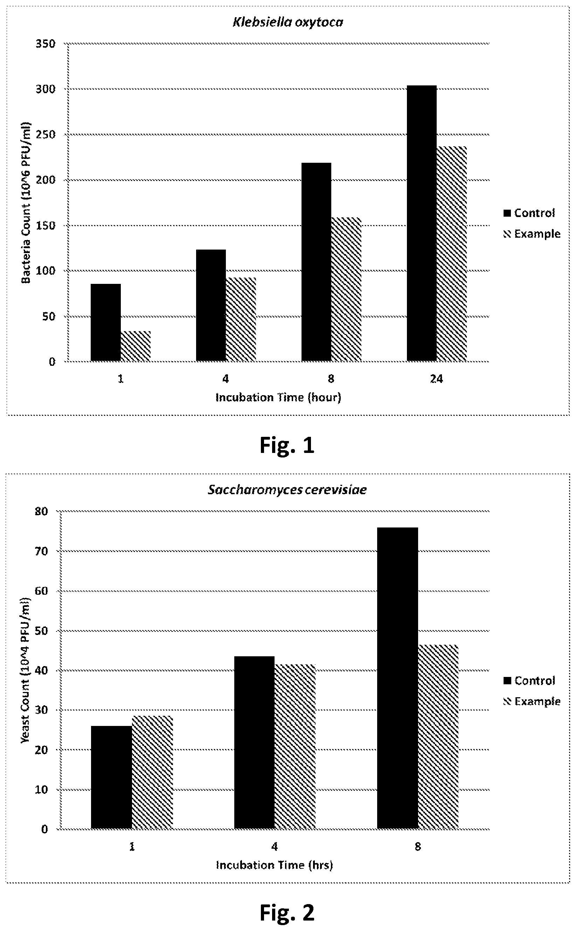

Autoclaved broth was made from about 30 g of tryptic soy broth (TSB) and about 1000 ml of deionized water. The autoclaved broth was inoculated with a pure colony of *Klebsiella oxytoca* and incubated for about 4 hours at a temperature from about 34 to about 38 degrees Celsius. After incubation, 1000 mg of the cerium (IV) oxide composition was charged into a flask containing about 100 ml of the inoculated broth solution, after which the flask was placed on an incubation shaker. Samples were taken after about 1, 4, 8, and 24 hours and, thereafter, diluted about 1,000,000 fold. About 100 µl of each of the diluted sample was spread on agar plates and incubated at a temperature from about 34 to about 38 degrees Celsius for from about 18 to about 24 hours, after which the number of colonies were then counted. The control consisted of about 100 ml of the inoculated broth solution charged to a flask. The flask was placed on an incubation shaker, after which samples were taken after about 1, 4, 8 and 24 hours. The samples were diluted, spread on agar plates and incubated according to the same procedures as the cerium (IV) oxide composition treated samples. The results of these tests are set forth below in Table 1 and Fig. 1. Fig. 1 shows the *Klebsiella oxytoca* bacteria with respect to the incubation time for a control and for the cerium (IV) oxide composition of the Example. Use of the cerium (IV) oxide composition leads to a lower bacteria count at 1, 4, 8, and 24 hour incubation times as compared to the control.

**Table 1**

| Incubation Time (hr) | Control (10⁶ PFU/ml) | Cerium (IV) Oxide Composition Example (10⁶ PFU/ml) |
|---|---|---|
| 1 | 86 | 34 |
| 4 | 124 | 93 |
| 8 | 219 | 159 |
| 24 | 304 | 237 |

### Yeast Removal Characteristics of the Cerium (IV) Oxide Composition

Autoclaved broth was made from about 30 g of tryptic soy broth (TSB) and about 1000 ml of deionized water. The autoclaved broth was inoculated with a pure colony of *Saccharomyces cerevisiae* and incubated for about 4 hours at about 34 to about 38 degrees Celsius. After incubation, about 1000 mg of the cerium (IV) oxide composition was placed into a flask containing 100 ml of the inoculated broth solution, after which the flask was placed on an incubated shaker. Samples were taken after about 1, 4, 8, and 24 hours and, thereafter, diluted about 1,000,000 fold. About 100 µl of each of the diluted sample was spread on agar plates and incubated at a temperature from about 34 to about 38 degrees Celsius for from about 18 to about 24 hours, after which the number of colonies were then counted. The control consisted of about 100 ml of the inoculated broth solution charged to a flask. The flask was placed on an incubation shaker, after which samples were taken after about 1, 4, 8 and 24 hours. The samples were diluted, spread on agar plates and incubated according to the same procedures as the cerium (IV) oxide composition treated samples. The results of these tests are set forth below in Table 2 and Fig. 2. Fig. 2 shows the *Saccharomyces cerevisiae* yeast count with respect to incubation time for a control and for the cerium (IV) oxide composition of the Example. While use of the cerium (IV) oxide composition leads to a slightly higher yeast count for an incubation time of 1 hour, it leads to lower yeast count for an incubation time of 4 hours, and a dramatically lower yeast count for an incubation time of 8 hours.

**Table 2**

| Incubation Time (hr) | Control (10⁶ PFU/ml) | Cerium (IV) Oxide Composition Example (10⁶ PFU/ml) |
|---|---|---|
| 1 | 26 | 29 |
| 4 | 44 | 42 |
| 8 | 76 | 47 |

### Algal Removal Characteristics of the Cerium (IV) Oxide Composition

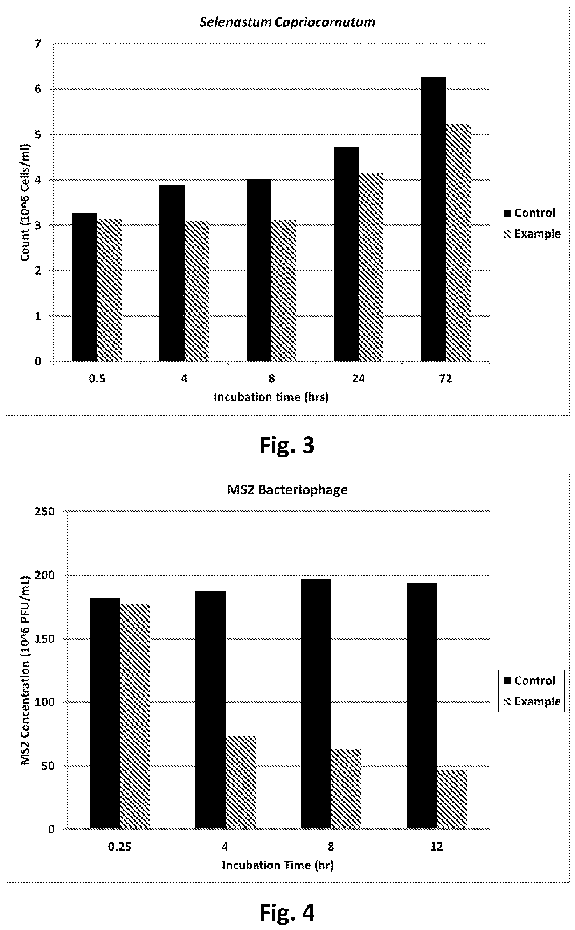

*Selenastum Capriocornutum* (UTEX) was cultured and about 100 ml of the culture was mixed with about 250 mg of the cerium (IV) oxide composition and about 50 ml of fresh Bristol Medium. The mixture was shaken at about 400 rpm and about 16 inches from incubation lights. A sample of about 100 µL was taken from the reactor at about 0.5, 4, 8, 24 and 48 hours. Each 100 µm sample was placed on a hemacytometer (HASSEUR Scientific) and observed under magnifications between about 300x and about 400x. Counts were taken for each visible cell within 0.015625 mm² grids, the depth of the sample in the hemacytometer is 0.1 mm. The control consisted of cultured medium incubated in the absence of the cerium (IV) oxide composition. The incubated control samples were taken and analyzed in the same manner as the samples incubated in the presence of the cerium (IV) oxide composition. The results of these tests are set forth below in Table 3 and Fig. 3. Fig. 3 shows the *Selenastum Capriocornutum* count with respect to incubation time for a control and for the cerium (IV) oxide composition of the Example. Use of the cerium (IV) oxide composition leads to a lower algae populations at 0.5, 1, 4, 8, 24 and 72 hour incubation times as compared to the control.

**Table 3**

| Incubation Time (hr) | Control (10⁶ PFU/ml) | Cerium (IV) Oxide Composition Example (10⁶ PFU/ml) |
|---|---|---|
| 0.5 | 3.3 | 3.1 |
| 4 | 3.9 | 3.1 |
| 8 | 4.0 | 3.1 |
| 24 | 4.7 | 4.2 |
| 72 | 6.3 | 5.2 |

### Viral Removal Characteristics of the Cerium (IV) Oxide Composition

About 500 mL of a buffered demand free (BFD) water (about 500 mL deionized water, about 285 mg Na₂HPO₄, and about 440 mg KH₂PO₄) was charged with about 1 ml of a MS2 bacteriophages stock solution; from which about 100 ml of the solution was taken and mixed with about 1000 mg of the cerium (IV) oxide composition. Thereafter, samples were taken at 0.25, 4, 8, and 12 hours, the each sample was diluted about 1,000,000 fold. *E. Coli* 15597 bacterial host was used to Assay the samples. About 100 µl of the *e. coli* solutions were spread on agar plates, after which the samples were incubated at a temperature from about 34 to about 38 degrees Celsius for about 18 to 24 hours. The control consisted of same buffered demand free water charged with the same MS2 bacteriophages, but in the absence of the cerium (IV) oxide composition. The control samples were taken and analyzed by the same procedures as the samples having the cerium (IV) oxide composition. After the incubation period, the number of colonies was then counted for each of the samples. The results of these tests are set forth below in Table 4 and Fig. 4. Fig. 4 shows the MS2 Bacteriophage concentration with respect to incubation time for a control and for the cerium (IV) oxide composition of the Example. While the cerium (IV) oxide composition of the Example and the Control show similar results for an incubation time of 0.25 hours, the cerium (IV) oxide composition of the Example dramatically reduces the population of the MS2 Bacteriophage as compared to the Control for 4, 8, and 12 hours.

**Table 4**

| Incubation Time (hr) | Control (10⁶ PFU/ml) | Cerium (IV) Oxide Composition Example (10⁶ PFU/ml) |
|---|---|---|
| 0.25 | 182 | 177 |
| 4 | 188 | 73 |
| 8 | 197 | 63 |
| 12 | 193 | 47 |

### Arsenic and Fluoride Removal

In order to test the arsenic adsorption characteristics of the cerium (IV) oxide composition the following equilibrium isotherm study was done. Test solutions containing arsenic in the form of arsenate or arsenite were prepared according to guidelines for NSF 53 Arsenic Removal water as specified in section 7.4.1.1.3 of NSF/ANSI 53 drinking water treatment units-health effects standards document. 20 milligrams of the cerium (IV) oxide composition, were placed in a sealed 500 milliliter polyethylene container and slurried with about 500 milliliters of the test solution containing arsenic at concentrations as described in Table 6. The resultant slurries were agitated by tumbling the containers for several hours. After agitation, the tap water was separated from the solids by filtration through a 0.45 micron syringe filter and sealed in 125 milliliter plastic sample bottles. The bottles were then sent to a certified drinking water analysis laboratory where the amount of arsenic in each liquid sample was determined by ICP mass spectroscopy. The results of these tests are set forth below in Tables 5 and 6.

**Table 5**

| Initial arsenic(V) concentration before treatment with cerium (IV) oxide composition (µg/L) | Final arsenic(V) concentration after treatment with cerium (IV) oxide composition (µg/L) | Arsenic removal capacity of cerium (IV) oxide composition (mg As/g CeO₂) |
|---|---|---|
| 20 | 2.6 | 0.88 |
| 75 | 19.3 | 2.83 |
| 140 | 52 | 4.46 |
| 290 | 156.7 | 6.76 |
| 470 | 310 | 7.92 |

**Table 6**

| Initial arsenic(III) concentration before treatment with cerium (IV) oxide composition (µg/L) | Final arsenic(III) concentration after treatment with cerium (IV) oxide composition (µg/L) | Arsenic removal capacity of cerium (IV) oxide composition (mg As/g CeO₂) |
|---|---|---|
| 19 | 2 | 0.86 |
| 77 | 2 | 3.81 |
| 140 | 3.1 | 6.94 |
| 270 | 23 | 12.52 |
| 440 | 85 | 17.57 |

In order to test the arsenic adsorption characteristics of the cerium (IV) oxide composition at different pH points the following study was done. Test solutions containing arsenic in the form of arsenate or arsenite were prepared at varying pH points according to guidelines for NSF 53 Arsenic Removal water as specified in section 7.4.1.1.3 of NSF/ANSI 53 drinking water treatment units-health effects standards document. 10 to 20 milligrams of the cerium (IV) oxide composition were placed in a sealed 500 milliliter polyethylene container and slurried with about 500 milliliters of the test solution at pH points as described in Tables 7 and 8. The resultant slurries were agitated by tumbling the containers for several hours. After agitation, the tap water was separated from the solids by filtration through a 0.2 micron syringe filter and sealed in 125 milliliter plastic sample bottles. The bottles were then sent to a certified drinking water analysis laboratory where the amount of arsenic in each liquid sample was determined by ICP mass spectroscopy. The results of these tests are set forth below in Tables 7 and 8.

**Table 7**

| pH of water | Initial arsenic(V) concentration before treatment with cerium (IV) oxide composition (µg/L) | Final arsenic(V) concentration after treatment with cerium (IV) oxide composition (µg/L) | Arsenic removal capacity of cerium (IV) oxide composition (mg As/g CeO₂) |
|---|---|---|---|
| 2.45 | 140 | 7.5 | 3.27 |
| 4.50 | 150 | 11 | 6.91 |
| 6.50 | 140 | 8 | 7.10 |
| 8.52 | 140 | 16 | 6.18 |
| 9.54 | 140 | 84 | 2.80 |
| 10.56 | 33 | 22 | 0.54 |

**Table 8**

| pH of water | Initial arsenic(III) concentration before treatment with cerium (IV) oxide composition (µg/L) | Final arsenic(III) concentration after treatment with cerium (IV) oxide composition (µg/L) | Arsenic removal capacity of cerium (IV) oxide composition (mg As/g CeO₂) |
|---|---|---|---|
| 2.43 | 130 | 45 | 4.27 |
| 4.42 | 130 | 8 | 6.02 |
| 6.43 | 130 | 7 | 6.21 |
| 8.38 | 130 | 8 | 6.17 |
| 9.54 | 130 | 9 | 6.06 |
| 10.71 | 69 | 11 | 2.92 |

In order to test the kinetics of arsenic adsorption of the said ceric oxide the following study was done. Test solutions containing arsenic (V) in the form of arsenate were prepared according to guidelines for NSF 53 Arsenic Removal water as specified in section 7.4.1.1.3 of NSF/ANSI 53 drinking water treatment units-health effects standards document. 10 milligrams of the ceric oxide, were placed in a sealed 500 milliliter polyethylene container and slurried with about 500 milliliters of the test solution at different pH points containing arsenic at concentrations as described in Tables 9 and 10. The resultant slurries were agitated by tumbling the containers for a set time given to each individual sample. After agitation, the tap water was separated from the solids by filtration through a 0.2 micron syringe filter and sealed in 125 milliliter plastic sample bottles. The bottles were then sent to a certified drinking water analysis laboratory where the amount of arsenic in each liquid sample was determined by ICP mass spectroscopy. The results of these tests are set forth below in Tables 9 and 10.

**Table 9**

| Equilibrium Time (min) | Initial arsenic(V) concentration before treatment with cerium (IV) oxide composition (µg/L) | Final arsenic(V) concentration after treatment with cerium (IV) oxide composition (µg/L) | Arsenic removal capacity of cerium (IV) oxide composition (mg As/g CeO₂) | Inverse of the arsenic removal capacity of cerium (IV) oxide composition (1/(mg As/g CeO₂)) |
|---|---|---|---|---|
| 18 | 100 | 38 | 3.13 | 26.32 |
| 34 | 100 | 27 | 3.76 | 37.04 |
| 77 | 100 | 18 | 4.18 | 55.56 |
| 139 | 100 | 11 | 4.54 | 90.91 |
| 228 | 100 | 6.9 | 4.66 | 144.93 |
| 475 | 100 | 4.1 | 4.99 | 243.90 |

**Table 10**

| Equilibrium Time (min) | Initial arsenic(III) concentration before treatment with cerium (IV) oxide composition (µg/L) | Final arsenic(III) concentration after treatment with cerium (IV) oxide composition (µg/L) | Arsenic removal capacity of cerium (IV) oxide composition (mg As/g CeO₂) | Inverse of the arsenic removal capacity of cerium (IV) oxide composition (1/(mg As/g CeO₂)) |
|---|---|---|---|---|
| 19 | 87 | 50 | 1.86 | 20 |
| 36 | 87 | 38 | 2.36 | 26.32 |
| 122 | 87 | 8 | 3.87 | 125 |
| 496 | 87 | 2 | 4.31 | 400.00 |

Test solutions containing Fluoride were prepared according to guidelines for NSF 53 Arsenic Removal water as specified in section 7.4.1.1.3 of NSF/ANSI 53 drinking water treatment units-health effects standards document. 500 milligrams of the cerium (IV) oxide composition of the Example were placed in a sealed 125 milliliter polyethylene container and slurried with about 50 milliliters of test solution with Fluoride concentrations as described in the Table. The resultant slurries were agitated by tumbling the containers for several hours. After agitation, the test solution was separated from the solids by filtration through a 0.45 micron syringe filter. The filtrate was sealed in 125 milliliter plastic sample bottles and sent to a certified drinking water analysis laboratory where the amount of arsenic in each filtrate was determined by ICP mass spectroscopy. The results of these tests are set forth below in Table 11.

**Table 11**

| Initial Fluoride concentration before treatment with cerium (IV) oxide composition (mg/L) | Final Fluoride concentration after treatment with cerium (IV) oxide composition (mg/L) | Fluoride removal capacity of with cerium (IV) oxide composition (mg F/g CeO₂) |
|---|---|---|
| 1.14 | 0.107 | 0.10 |
| 5.1 | 0.263 | 0.48 |
| 10.7 | 0.713 | 1.00 |
| 20.4 | 0.2533 | 1.80 |
| 48 | 15.600 | 3.21 |

Test solutions containing Fluoride were prepared according to guidelines for NSF 53 Arsenic Removal water as specified in section 7.4.1.1.3 of NSF/ANSI 53 drinking water treatment units-health effects standards document. 500 milligrams of the cerium (IV) oxide composition of the Example were placed in a sealed 125 milliliter polyethylene container and slurried with about 50 milliliters of test solution at different pH points as described in the Table. The resultant slurries were agitated by tumbling the containers for several hours. After agitation, the test solution was separated from the solids by filtration through a 0.45 micron syringe filter. The filtrate was sealed in 125 milliliter plastic sample bottles and sent to a certified drinking water analysis laboratory where the amount of arsenic in each filtrate was determined by ICP mass spectroscopy. The results of these tests are set forth below in Table 12.

**Table 12**

| pH of Water | Final Fluoride concentration after treatment with cerium (IV) oxide composition (µg/L) | Fluoride removal capacity of cerium (IV) oxide composition (mg As/g CeO₂) |
|---|---|---|
| 2.53 | 0.167 | 6.82 |
| 4.53 | 1.300 | 6.45 |
| 6.47 | 2.227 | 5.10 |
| 8.63 | 3.133 | 4.22 |
| 9.46 | 9.200 | 6.06 |
| 10.5 | 6.050 | 0.95 |

### COMPARATIVE EXAMPLES

The comparative examples use an oxide of cerium (IV) prepared calcining Ce₂(CO₃)₃·6H₂O in a muffle furnace for 2 hours. The oxide of cerium is represented by the chemical formula CeO₂ and the cerium has an oxidation state of +4. The oxide of cerium used in the comparative examples has a Zeta potential of about 16 mV at pH 7, an iso-electric point of about pH 8.8, about 0.02 acidic sites/kg as measured by zeta-potential titration, a particle size D₁₀ of about 4 µm, particle size D₅₀ of about 30µum, a particle size D₉₀ of about 90 µm, and a crystallite size of about 19 nm.

### Bacterial Removal Characteristics of an Oxide of Cerium (IV)

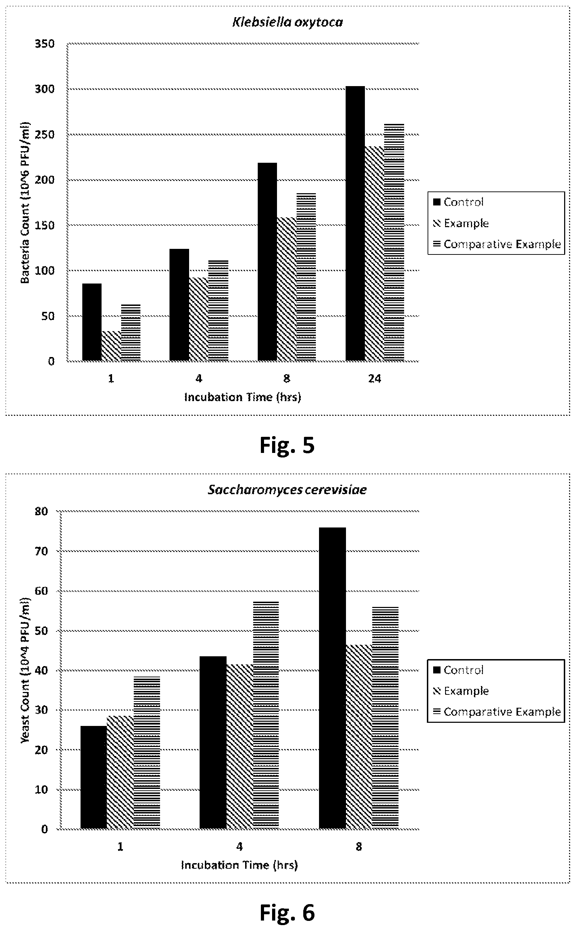

Autoclaved broth was made from about 30 g of tryptic soy broth (TSB) and about 1000 ml of deionized water. The autoclaved broth was inoculated with a pure colony of *Klebsiella oxytoca* and incubated for about 4 hours at a temperature from about 34 to about 38 degrees Celsius. After incubation, 1000 mg of the oxide of cerium (IV) was charged into a flask containing about 100 ml of the inoculated broth solution, after which the flask was placed on an incubation shaker. Samples were taken after about 1, 4, 8, and 24 hours and, thereafter, diluted about 1,000,000 fold. About 100 µl of each of the diluted sample was spread on agar plates and incubated at a temperature from about 34 to about 38 degrees Celsius for from about 18 to about 24 hours, after which the number of colonies were then counted. The results of these tests are set forth below in Table 13 and Fig. 5. Fig. 5 shows the *Klebsiella oxytoca* count with respect to incubation time for a control, the cerium (IV) oxide composition of the Example, and for an oxide of cerium (IV) of the prior art (Comparative Example). Compared to the control and Comparative Example, the cerium (IV) oxide composition of Example leads to a lower bacteria count at every incubation time.

**Table 13**

| Incubation Time (hr) | Control (10⁶ PFU/ml) | Cerium (IV) Oxide Composition Example (10⁶ PFU/ml) | Oxide of Cerium (IV) Comparative Example (10⁶ PFU/ml) |
|---|---|---|---|
| 1 | 86 | 34 | 64 |
| 4 | 124 | 93 | 112 |
| 8 | 219 | 159 | 185 |
| 24 | 304 | 237 | 263 |

### Yeast Removal Characteristics of an Oxide of Cerium (IV)

Autoclaved broth was made from about 30 g of tryptic soy broth (TSB) and about 1000 ml of deionized water. The autoclaved broth was inoculated with a pure colony of *Saccharomyces cerevisiae* and incubated for about 4 hours at about 34 to about 38 degrees Celsius. After incubation, about 1000 mg of the oxide of cerium (IV) was placed into a flask containing 100 ml of the inoculated broth solution, after which the flask was placed on an incubated shaker. Samples were taken after about 1, 4, 8, and 24 hours and, thereafter, diluted about 1,000,000 fold. About 100 µl of each of the diluted sample was spread on agar plates and incubated at a temperature from about 34 to about 38 degrees Celsius for from about 18 to about 24 hours, after which the number of colonies were then counted. The results of these tests are set forth below in Table 14 and Fig. 6. Fig. 6 shows the *Saccharomyces cerevisiae* count with respect to incubation time for a control, the cerium (IV) oxide composition of the Example, and the oxide of cerium (IV) of the Comparative Example. For an incubation time of 1 hour, the control leads to a lower yeast count compared to both the cerium (IV) oxide composition of Example and the oxide of cerium (IV) of the Comparative Example. However, for an incubation time of 4 hours, while the control still leads to a lower yeast count than Comparative Example, the cerium (IV) oxide composition of Example leads to a lower yeast count than both the control and Comparative Example. Lastly, for an incubation time of 8 hours, both the cerium (IV) oxide composition of the Example and oxide of cerium (IV) of the Comparative Example outperform the control, while the cerium (IV) oxide composition of Example leads to a lower yeast count than the Comparative Example.

**Table 14**

| Incubation Time (hr) | Control (10⁶ PFU/ml) | Cerium (IV) Oxide Composition Example (10⁶ PFU/ml) | Oxide of Cerium (IV) Comparative Example (10⁶ PFU/ml) |
|---|---|---|---|
| 1 | 26 | 29 | 39 |
| 4 | 44 | 42 | 58 |
| 8 | 76 | 47 | 56 |

### Algal Removal Characteristics of an Oxide of Cerium (IV)

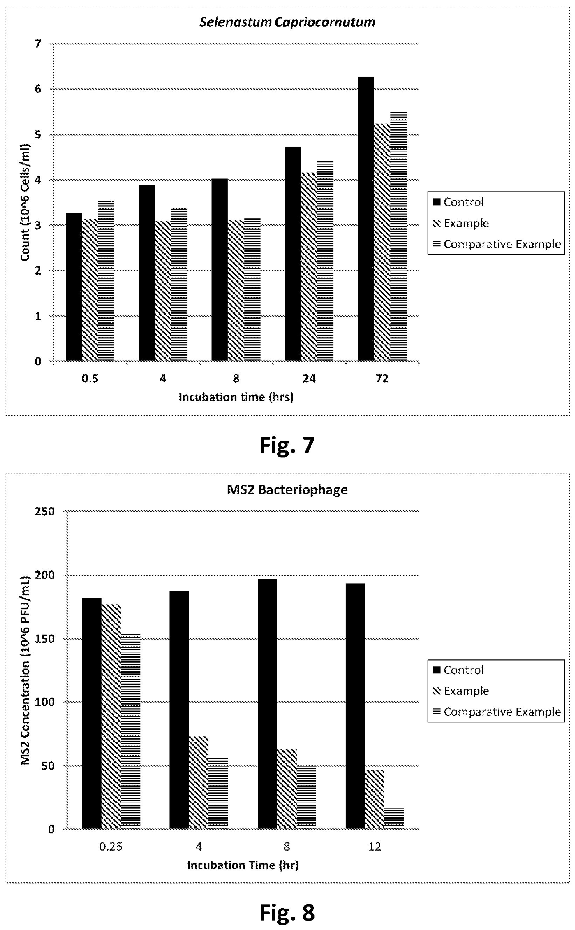

*Selenastum Capriocornutum* (UTEX) was cultured and about 100 ml of the culture was mixed with about 250 mg of an oxide of cerium (IV) and about 50 ml of fresh Bristol Medium. The mixture was shaken at about 400 rpm and about 16 inches from incubation lights. A sample of about 100 µL was taken from the reactor at about 0.5, 4, 8, 24 and 48 hours. Each 100 µm sample was placed on a hemacytometer (HASSEUR Scientific) and observed under magnifications between about 300x and about 400x. Counts were taken for each visible cell within 0.015625 mm² grids, the depth of the sample in the hemacytometer is 0.1 mm. The results of these tests are set forth below in Table 15 and Fig. 7. Fig. 7 shows the *Selenastum Capriocornutum* count with respect to incubation time with respect to a control, the cerium (IV) oxide composition of the Example, and Comparative Example (an oxide of cerium (IV) of the prior art). For every incubation time, the use of the cerium (IV) oxide composition of the Example leads to a lower algae count compared to both the control and Comparative Example.

**Table 15**

| Incubation Time (hr) | Control (10⁶ PFU/ml) | Cerium (IV) Oxide Composition Example (10⁶ PFU/ml) | Oxide of Cerium (IV) Comparative Example (10⁶ PFU/ml) |
|---|---|---|---|
| 0.5 | 3.3 | 3.1 | 3.5 |
| 4 | 3.9 | 3.1 | 3.4 |
| 8 | 4.0 | 3.1 | 3.2 |
| 24 | 4.7 | 4.2 | 4.4 |
| 72 | 6.3 | 5.2 | 5.5 |

### Viral Removal Characteristics of an Oxide of Cerium (IV)

About 500 mL of a buffered demand free (BFD) water (about 500 mL deionized water, about 285 mg Na₂HPO₄, and about 440 mg KH₂PO₄) was charged with about 1 ml of a MS2 bacteriophages stock solution; from which about 100 ml of the solution was taken and mixed with about 1000 mg of the oxide of cerium (IV). Thereafter, samples were taken at 0.25, 4, 8, and 12 hours, the each sample was diluted about 1,000,000 fold. *E. Coli* 15597 bacterial host was used to Assay the samples. About 100 µl of the *e. coli* solutions were spread on agar plates, after which the samples were incubated at a temperature from about 34 to about 38 degrees Celsius for about 18 to 24 hours. After the incubation period, the number of colonies was then counted for each of the samples. The results of these tests are set forth below in Table 16 and Fig. 8. Fig. 8 shows the MS2 Bacteriophage concentration with respect to incubation time for a control, the cerium (IV) oxide composition of the Example, and the Comparative Example (an oxide of cerium (IV) of the prior art). While the cerium (IV) oxide composition of the Example outperforms the control in effectively lowering the virus count at every incubation time, and significantly lowers the viral count compared with the control at incubation times of 4, 8, and 12 hours, the cerium (IV) oxide composition of Example does not lower the count as effectively as the Comparative Example.

**Table 16**

| Incubation Time (hr) | Control (10⁶ PFU/ml) | Cerium (IV) Oxide Composition Example (10⁶ PFU/ml) | Oxide of Cerium (IV) Comparative Example (10⁶ PFU/ml) |
|---|---|---|---|
| 0.25 | 182 | 177 | 154 |
| 4 | 188 | 73 | 56 |
| 8 | 197 | 63 | 51 |
| 12 | 193 | 47 | 17 |

### Zeta Potential and Particle Size Distribution

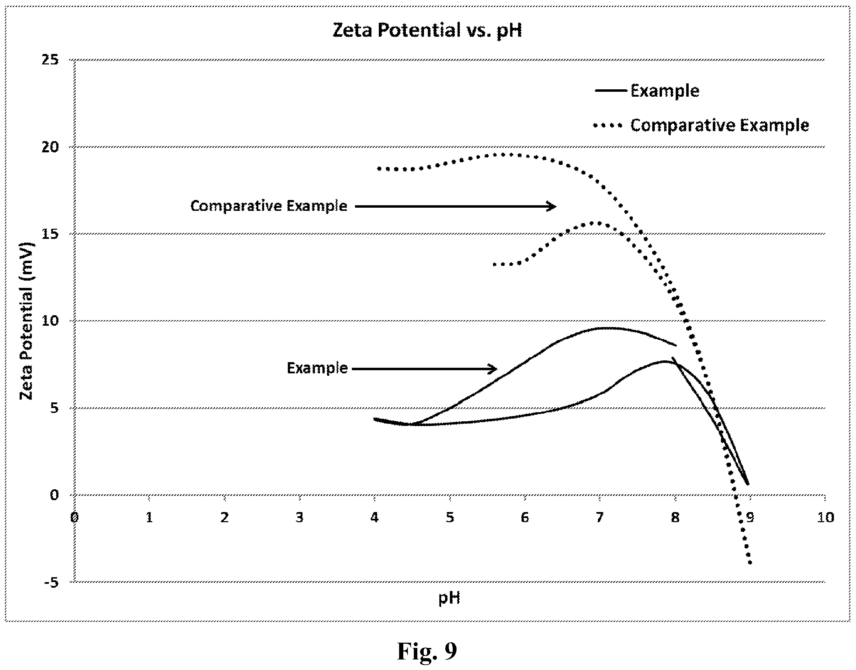

Fig. 9 shows the zeta potential for both the Example and the Comparative Example as a function of pH. The zeta potential of the cerium (IV) oxide composition of the Example is higher from a pH of about 4 until a pH of about 8.5. For a pH of above about 8.5, the Comparative Example has a larger zeta potential.

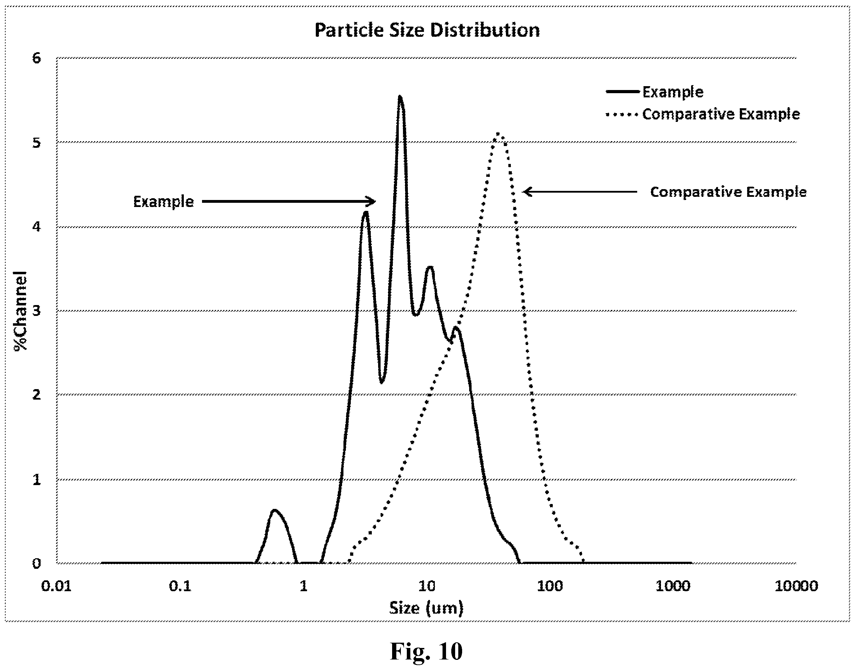

Fig. 10 shows the particle size distribution for both the Example and the Comparative Example. The particle size distribution of the Example is much less uniform than that of the Comparative Example, and the cerium (IV) oxide composition of the Example also has a smaller average particle size than the Comparative Example.

### Arsenic and Fluoride Removal

Test solutions containing arsenic(V) were prepared according to guidelines for NSF 53 Arsenic Removal water as specified in section 7.4.1.1.3 of NSF/ANSI 53 drinking water treatment units-health effects standards document. 20 milligrams of commercially available oxide of cerium (IV) (CeO₂ prepared by calcining Ce₂(CO₃)₃·6H₂O and having a Zeta potential of about 16 mV at pH 7, an iso-electric point of about pH 8.8, a particle size D₁₀ of about 4 um, particle size D₅₀ of about 30 um, a particle size D₉₀ of about 90 um, and a crystallite size of about 19 nm. in a muffle furnace for 2 hours), were placed in a sealed 500 milliliter polyethylene container and slurried with about 500 milliliters of an arsenic test solution at concentrations as described in Tables 1-8. The resultant slurries were agitated by tumbling the containers for several hours. After agitation, the test solution was separated from the solids by filtration through a 0.45 micron syringe filter. The filtrate was sealed in 125 milliliter plastic sample bottles and sent to a certified drinking water analysis laboratory where the amount of arsenic in each filtrate was determined by ICP mass spectroscopy. The results of these tests are set forth below in Tables 5-12.

**Table 17**

| Initial Arsenic(V) concentration before treatment with ceric oxide (µg/L) | Final Arsenic(V) concentration after treatment with ceric oxide (µg/L) | Arsenic removal capacity of ceric oxide (mg As/g CeO₂ |
|---|---|---|
| 19 | 15 | 0.20 |
| 78 | 65 | 0.64 |
| 190 | 170 | 1.00 |
| 290 | 260 | 1.48 |
| 480 | 443 | 1.84 |

**Table 18**

| Initial arsenic(III) concentration before treatment with an oxide of cerium (IV) of the prior art (µg/L) | Final arsenic(III) concentration after treatment with oxide of cerium (IV) of the prior art (µg/L) | Arsenic removal capacity of an oxide of cerium (III) of the prior art (mg As/g CeO₂) |
|---|---|---|
| 20 | 2.9 | 0.85 |
| 79 | 13 | 3.25 |
| 140 | 32 | 5.42 |
| 270 | 92 | 8.78 |
| 450 | 200 | 12.54 |

**Table 19**

| pH of water | Initial arsenic(V) concentration before treatment with cerium (IV) oxide composition (µg/L) | Final arsenic(V) concentration after treatment with cerium (IV) oxide composition (µg/L) | Arsenic removal capacity of cerium (IV) oxide composition (mg As/g CeO₂) |
|---|---|---|---|
| 2.45 | 140 | 39 | 5.15 |
| 4.50 | 150 | 12 | 6.89 |
| 6.50 | 140 | 46 | 4.75 |
| 8.52 | 140 | 110 | 1.50 |
| 9.54 | 140 | 127 | 0.67 |
| 10.56 | 33 | 25 | 0.38 |

**Table 20**

| pH of water | Initial arsenic(III) concentration before treatment with cerium (IV) oxide composition (µg/L) | Final arsenic(III) concentration after treatment with cerium (IV) oxide composition (µg/L) | Arsenic removal capacity of cerium (IV) oxide composition (mg As/g CeO₂) |
|---|---|---|---|
| 2.43 | 130 | 22 | 5.23 |
| 4.42 | 130 | 5 | 6.29 |
| 6.43 | 130 | 14 | 5.73 |
| 8.38 | 130 | 35 | 4.61 |
| 9.54 | 130 | 61 | 3.50 |
| 10.71 | 69 | 36 | 1.66 |

**Table 21**

| Equilibrium Time (min) | Initial arsenic(V) concentration before treatment with cerium (IV) oxide composition (µg/L) | Final arsenic(V) concentration after treatment with cerium (IV) oxide composition (µg/L) | Arsenic removal capacity of cerium (IV) oxide composition (mg As/g CeO₂) | Inverse of the arsenic removal capacity of cerium (IV) oxide composition (1/(mg As/g CeO₂)) |
|---|---|---|---|---|
| 19 | 100 | 95 | 0.25 | 10.53 |
| 34 | 100 | 92 | 0.41 | 10.87 |
| 68 | 100 | 87 | 0.65 | 11.49 |
| 129 | 100 | 82 | 0.88 | 12.20 |
| 222 | 100 | 76 | 1.21 | 13.16 |
| 470 | 100 | 68 | 1.60 | 14.49 |

**Table 22**

| Equilibrium Time (min) | Initial arsenic(III) concentration before treatment with cerium (IV) oxide composition (µg/L) | Final arsenic(III) concentration after treatment with cerium (IV) oxide composition (µg/L) | Arsenic removal capacity of cerium (IV) oxide composition (mg As/g CeO₂) | Inverse of the arsenic removal capacity of cerium (IV) oxide composition (1/(mg As/g CeO₂)) |
|---|---|---|---|---|
| 19 | 87 | 78 | 0.45 | 12.82 |
| 35 | 87 | 80 | 0.36 | 12.50 |
| 68 | 87 | 66 | 1.00 | 15.15 |
| 122 | 87 | 59 | 1.47 | 16.95 |
| 257 | 87 | 52 | 1.68 | 19.23 |
| 485 | 87 | 49 | 1.88 | 20.41 |

**Table 23**

| Initial Fluoride concentration before treatment with cerium (IV) oxide composition (µg/L) | Final Fluoride concentration after treatment with cerium (IV) oxide composition (µg/L) | Fluoride removal capacity of cerium (IV) oxide composition (mg F/g CeO₂) |
|---|---|---|
| 1.14 | 0.107 | 0.10 |
| 5.1 | 0.263 | 0.48 |
| 10.7 | 0.713 | 1.00 |
| 20.4 | 0.2533 | 1.80 |
| 48 | 15.600 | 3.21 |

**Table 24**

| pH of Water | Final Fluoride concentration after treatment with with cerium (IV) oxide composition (µg/L) | Fluoride removal capacity of cerium (IV) oxide composition (mg F/g CeO₂) |
|---|---|---|
| 2.53 | 0.167 | 6.82 |
| 4.53 | 1.300 | 6.45 |
| 6.47 | 2.227 | 5.10 |
| 8.63 | 3.133 | 4.22 |
| 9.46 | 9.200 | 6.06 |
| 10.5 | 6.050 | 0.95 |

The arsenic (III) and arsenic (V) removal data as depicted in Tables 5-10 for the cerium (IV) oxide composition and Tables 17-22 for the oxide of cerium (IV) of the prior art clearly show that the cerium (IV) composition has expected properties towards arsenic (III) and arsenic (IV). In other words, a person of ordinary skill in the art of rare earths and/or water treatment chemistry would not expect the cerium (IV) oxide composition of the present disclosure to remove arsenic from an aqueous stream differently than the oxide of cerium (IV) of the prior art. Furthermore, the cerium (IV) oxide composition remove fluoride from an aqueous differently than the oxide of cerium (IV) of the prior art, as depicted in Tables 11, 12, 22 and 23. It has also been found that these surprising and unexpected properties are also applicable to biological contaminant removal as shown in Tables 1-4 and 13-16.

Not wishing to be bound by any theory, the aforementioned examples illustrate that the cerium (IV) oxide composition embodied in the present disclosure provides for much better biological contaminant removal performance owing to its unique material characteristics.

The present disclosure, in various aspects, embodiments, and configurations, includes components, methods, processes, systems and/or apparatus substantially as depicted and described herein, including various aspects, embodiments, configurations, sub-combinations, and subsets thereof. Those of skill in the art will understand how to make and use the various aspects, aspects, embodiments, and configurations, after understanding the present disclosure.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more, aspects, embodiments, and configurations for the purpose of streamlining the disclosure.

## Claims

1. A method for removing biological contaminants from an aqueous stream, comprising:
contacting a cerium (IV) oxide composition with a biological contaminant-containing aqueous stream, wherein the following are true:
(i) the cerium (IV) oxide composition has a zeta potential at pH 7 of no more than 30 mV and of more than 1 mV;
(ii) the cerium (IV) oxide composition has a particle size D₁₀ of more than 0.5 µm and no more than 7 µm;
(iii) the cerium (IV) oxide composition has a particle size D₅₀ of more than 2 µm and no more than 20 µm;
(iv) the cerium (IV) oxide composition has a particle size D₉₀ of more than 12 µm and no more than 50 µm;
(v) the cerium (IV) oxide composition has a crystallite size of more than 1 nm and no more than 22 nm; and
(vi) the cerium (IV) oxide composition has an acidic site concentration of more than 0.0001 acidic sites/kg and no more than 0.020 acidic sites/kg; and
wherein the contacting of the cerium (IV) oxide composition with the biological contaminant-containing aqueous stream removes some of the biological contaminant from the biological contaminant-containing aqueous stream.

2. The method of claim 1, wherein the biological contaminant is selected from the group consisting of bacteria, yeasts, algae, and viruses.

3. The method of claim 1, wherein the biological contaminant is one selected from the group of *Klebsiella oxytoca, Saccharomyces cerevisiae,* Selenastum *capriocornutum,* and MS2 bacteriophage.

4. The method of claim 1, wherein one or more of the following is true:
(a) the zeta potential at pH 7 is from 7.5 to 12.5 mV;
(b) the particle size D₁₀ is from 1 to 3 µm;
(c) the particle size D₅₀ is from 7.5 to 10.5 µm;
(d) the particle size D₉₀ is from 20 to 30 µm; and
(e) the crystallite size is from 7.5 to 12.5 nm.

5. A biological contaminant removal device, comprising:
an inlet to receive an aqueous stream having a first level of a biological contaminant;
a contacting chamber, in fluid communication with the inlet and containing a cerium (IV) oxide composition to contact the aqueous stream, wherein the following (i)-(vi) are true:
(i) the cerium (IV) oxide composition has a zeta potential at pH 7 of no more than 30 mV and of more than 1 mV;
(ii) the cerium (IV) oxide composition has a particle size D₁₀ of more than 0.5 µm and no more than 7 µm;
(iii) the cerium (IV) oxide composition has a particle size D₅₀ of more than 2 µm and no more than 20 µm;
(iv) the cerium (IV) oxide composition has a particle size D₉₀ of more than 12 µm and no more than 50 µm;
(v) the cerium (IV) oxide composition has a crystallite size of more than 1 nm and no more than 22 nm; and
(vi) the cerium (IV) oxide composition has an acidic site concentration of more than 0.0001 acidic sites/kg and no more than 0.020 acidic sites/kg; and
wherein the aqueous stream has the first level of biological contaminant prior to contacting the cerium (IV) oxide composition and a second level of biological contaminant after contacting the cerium (IV) oxide, wherein the first level of biological contaminant is greater than the second level of biological contaminant, and
an outlet in fluid communication with the contacting chamber to output the aqueous stream having the second level of biological contaminant.

6. The device of claim 5, wherein the biological contaminant is selected from the group consisting of bacteria, yeasts, algae, and viruses.

7. The device of claim 5, wherein the biological contaminant is one selected from the group of Klebsiella oxytoca, Saccharomyces cerevisiae, Selenastum capriocomutum, and bacteriophage MS2.

8. The device of claim 5, wherein one or more of the following is true:
(a) the zeta potential at pH 7 is from 7.5 to 12.5 mV;
(b) the particle size D₁₀ is from 1 to 3 µm;
(c) the particle size D₅₀ is from 7.5 to 10.5 µm;
(d) the particle size D₉₀ is from 20 to 30 µm; and
(e) the crystallite size is from 7.5 to 12.5 nm.

## Patentansprüche

1. Ein Verfahren zum Entfernen biologischer Verunreinigungen aus einem wässrigen Strom, umfassend:
In-Kontakt-Bringen einer Cer(IV)-oxidzusammensetzung mit einem eine biologische Verunreinigung enthaltenden wässrigen Strom, wobei Folgendes zutrifft:
(i) die Cer(IV)-oxidzusammensetzung hat bei pH 7 ein Zetapotential von nicht mehr als 30 mV und von mehr als 1 mV;
(ii) die Cer(IV)-oxidzusammensetzung hat eine Partikelgröße D₁₀ von mehr als 0,5 µm und nicht mehr als 7 µm;
(iii) die Cer(IV)-oxidzusammensetzung hat eine Partikelgröße D₅₀ von mehr als 2 µm und nicht mehr als 20 µm;
(iv) die Cer(IV)-oxidzusammensetzung hat eine Partikelgröße D₉₀ von mehr als 12 µm und nicht mehr als 50 µm;
(v) die Cer(IV)-oxidzusammensetzung hat eine Kristallitgröße von mehr als 1 nm und nicht mehr als 22 nm; und
(vi) die Cer(IV)-oxidzusammensetzung hat eine Konzentration an aziden Zentren von mehr als 0,0001 aziden Zentren/kg und nicht mehr als 0,020 aziden Zentren/kg; und
wobei das In-Kontakt-Bringen der Cer(IV)-oxidzusammensetzung mit dem eine biologische Verunreinigung enthaltenden wässrigen Strom einen Teil der biologischen Verunreinigung aus dem eine biologische Verunreinigung enthaltenden wässrigen Strom entfernt.

2. Das Verfahren nach Anspruch 1, wobei die biologische Verunreinigung aus der Gruppe bestehend aus Bakterien, Hefen, Algen und Viren ausgewählt ist.

3. Das Verfahren nach Anspruch 1, wobei die biologische Verunreinigung aus der Gruppe aus *Klebsiella oxytoca, Saccharomyces cerevisiae,* Selenastum *capriocornutum* und MS2 Bakteriophage ausgewählt ist.

4. Das Verfahren nach Anspruch 1, wobei einer oder mehrere der folgenden Aussagen zutreffen:
(a) das Zetapotenzial bei pH 7 liegt zwischen 7,5 und 12,5 mV;
(b) die Partikelgröße D₁₀ liegt zwischen 1 und 3 µm;
(c) die Partikelgröße D₅₀ liegt zwischen 7,5 und 10,5 µm;
(d) die Partikelgröße D₉₀ liegt zwischen 20 und 30 µm; und
(e) die Kristallitgröße liegt zwischen 7,5 und 12,5 nm.

5. Eine Vorrichtung zum Entfernen biologischer Verunreinigungen, umfassend:
einen Einlass zur Aufnahme eines wässrigen Stroms mit einem ersten Gehalt einer biologischen Verunreinigung; eine Kontaktkammer, die in fluidischer Verbindung mit dem Einlass steht und eine Cer(IV)-oxidzusammensetzung zum Kontaktieren des wässrigen Stroms enthält, wobei die folgenden Punkte (i)-(vi) zutreffen:
(i) die Cer(IV)-oxidzusammensetzung hat bei pH 7 ein Zetapotential von nicht mehr als 30 mV und von mehr als 1 mV;
(ii) die Cer(IV)-oxidzusammensetzung hat eine Partikelgröße D₁₀ von mehr als 0,5 µm und nicht mehr als 7 µm;
(iii) die Cer(IV)-oxidzusammensetzung hat eine Partikelgröße D₅₀ von mehr als 2 µm und nicht mehr als 20 µm;
(iv) die Cer(IV)-oxidzusammensetzung hat eine Partikelgröße D₉₀ von mehr als 12 µm und nicht mehr als 50 µm;
(v) die Cer(IV)-oxidzusammensetzung hat eine Kristallitgröße von mehr als 1 nm und nicht mehr als 22 nm; und
(vi) die Cer(IV)-oxidzusammensetzung hat eine Konzentration an aziden Zentren von mehr als 0,0001 aziden Zentren/kg und nicht mehr als 0,020 aziden Zentren/kg; und
wobei der wässrige Strom den ersten Gehalt der biologischen Verunreinigung vor dem Kontaktieren der Cer(IV)-oxidzusammensetzung, und einen zweiten Gehalt der biologischen Verunreinigung nach dem Kontaktieren der Cer(IV)-oxidzusammensetzung aufweist, wobei der erste Gehalt der biologischen Verunreinigung größer ist als der zweite Gehalt der biologischen Verunreinigung, und
einen Auslass in fluidischer Verbindung mit der Kontaktkammer, um den wässrigen Strom mit dem zweiten Gehalt der biologischen Verunreinigung auszugeben.

6. Die Vorrichtung nach Anspruch 5, wobei die biologische Verunreinigung aus der aus Bakterien, Hefen, Algen und Viren bestehenden Gruppe ausgewählt ist.

7. Die Vorrichtung nach Anspruch 5, wobei die biologische Verunreinigung aus der Gruppe der Klebsiella oxytoca, Saccharomyces cerevisiae, Selenastum capriocornutum und Bakteriophage MS2 ausgewählt ist.

8. Die Vorrichtung nach Anspruch 5, wobei einer oder mehrere der folgenden Punkte zutreffen:
(a) das Zetapotenzial bei pH 7 liegt zwischen 7,5 und 12,5 mV;
(b) die Partikelgröße D₁₀ liegt zwischen 1 und 3 µm;
(c) die Partikelgröße D₅₀ liegt zwischen 7,5 und 10,5 µm;
(d) die Partikelgröße D₉₀ liegt zwischen 20 und 30 µm; und
(e) die Kristallitgröße liegt zwischen 7,5 und 12,5 nm.

## Revendications

1. Procédé pour éliminer des contaminants biologiques d'un courant aqueux, comprenant les étapes consistant à :
mettre en contact une composition d'oxyde de cérium (IV) avec un courant aqueux contenant des contaminants biologiques, où les points suivants sont avérés :
(i) la composition d'oxyde de cérium (IV) a un potentiel zêta à pH 7 inférieur ou égal à 30 mV et supérieur à 1 mV ;
(ii) la composition d'oxyde de cérium (IV) a une taille de particules D₁₀ supérieure à 0,5 µm et inférieure ou égale à 7 µm ;
(iii) la composition d'oxyde de cérium (IV) a une taille de particules D₅₀ supérieure à 2 µm et inférieure ou égale à 20 µm ;
(iv) la composition d'oxyde de cérium (IV) a une taille de particules D₉₀ supérieure à 12 µm et inférieure ou égale à 50 µm ;
(v) la composition d'oxyde de cérium (IV) a une taille de cristallites supérieure à 1 nm et inférieure ou égale à 22 nm ; et
(vi) la composition d'oxyde de cérium (IV) a une concentration en sites acides supérieure à 0,0001 sites acides/kg et inférieure ou égale à 0,020 sites acides/kg ; et
la mise en contact de la composition d'oxyde de cérium (IV) avec le courant aqueux contenant des contaminants biologiques éliminant une partie des contaminants biologiques du courant aqueux contenant des contaminants biologiques.

2. Procédé selon la revendication 1, dans lequel le contaminant biologique est choisi dans le groupe constitué des bactéries, des levures, des algues et des virus.

3. Procédé selon la revendication 1, dans lequel le contaminant biologique est un contaminant choisi dans le groupe comprenant *Klebsiella oxytoca, Saccharomyces cerevisiae, Selenastum capriocornutum,* et le bactériophage MS2.

4. Procédé selon la revendication 1, dans lequel l'un ou plusieurs des points suivants sont avérés :
a) le potentiel zêta à pH 7 est compris entre 7,5 et 12,5 mV ;
b) la taille de particules D₁₀ est comprise entre 1 et 3 µm ;
c) la taille de particules D₅₀ est comprise entre 7,5 et 10,5 µm ;
d) la taille de particules D₉₀ est comprise entre 20 et 30 µm ; et
e) la taille des cristallites est comprise entre 7,5 et 12,5 nm.

5. Dispositif d'élimination de contaminants biologiques, comprenant :
une entrée pour recevoir un courant aqueux ayant un premier niveau d'un contaminant biologique ;
une chambre de contact, en communication fluidique avec l'entrée et contenant une composition d'oxyde de cérium (IV) pour entrer en contact avec le courant aqueux, où les points (i)-(vi) suivants sont avérés :
(i) la composition d'oxyde de cérium (IV) a un potentiel zêta à pH 7 inférieur ou égal à 30 mV et supérieur à 1 mV ;
(ii) la composition d'oxyde de cérium (IV) a une taille de particules D₁₀ supérieure à 0,5 µm et inférieure ou égale à 7 µm ;
(iii) la composition d'oxyde de cérium (IV) a une taille de particules D₅₀ supérieure à 2 µm et inférieure ou égale à 20 µm ;
(iv) la composition d'oxyde de cérium (IV) a une taille de particules D₉₀ supérieure à 12 µm et inférieure ou égale à 50 µm ;
(v) la composition d'oxyde de cérium (IV) a une taille de cristallites supérieure à 1 nm et inférieure ou égale à 22 nm ; et
(vi) la composition d'oxyde de cérium (IV) a une concentration en sites acides supérieure à 0,0001 sites acides/kg et inférieure ou égale à 0,020 sites acides/kg ; et
le courant aqueux ayant le premier niveau de contaminant biologique avant d'être en contact avec la composition d'oxyde de cérium (IV) et un deuxième niveau de contaminant biologique après avoir été en contact de l'oxyde de cérium (IV), le premier niveau de contaminant biologique étant supérieur au deuxième niveau de contaminant biologique, et
une sortie en communication fluidique avec la chambre de contact pour évacuer le courant aqueux ayant le deuxième niveau de contaminant biologique.

6. Dispositif selon la revendication 5, dans lequel le contaminant biologique est choisi dans le groupe constitué des bactéries, des levures, des algues et des virus.

7. Dispositif selon la revendication 5, dans lequel le contaminant biologique est un contaminant choisi dans le groupe comprenant *Klebsiella oxytoca, Saccharomyces cerevisiae, Selenastum capriocornutum* et le bactériophage MS2.

8. Dispositif selon la revendication 5, dans lequel un ou plusieurs des points suivants sont avérés :
a) le potentiel zêta à pH 7 est compris entre 7,5 et 12,5 mV ;
b) la taille de particules D₁₀ est comprise entre 1 et 3 µm ;
c) la taille de particules D₅₀ est comprise entre 7,5 et 10,5 µm ;
d) la taille de particules D₉₀ est comprise entre 20 et 30 µm ; et
e) la taille des cristallites est comprise entre 7,5 et 12. 5 nm.
